# EUROPEAN PATENT APPLICATION

(11) **EP 3 020 806 A1**
(43) Date of publication of application: **18.05.2016**
(21) Application number: 14823622.7
(22) Date of filing: 11.07.2014
(51) Int. Cl.: C12N 15/00, A61K 39/00, A61P 35/00, A61P 43/00, C07K 7/06, C07K 16/30, C12N 15/09

(54) **TUMOR ANTIGEN PEPTIDE**

(30) Priority: 12.07.2013 JP 2013146849
(71) Applicant: Sumitomo Dainippon Pharma Co., Ltd., Osaka-shi Osaka 541-8524 (JP); Sapporo Medical University, Sapporo-shi, Hokkaido 060-8556 (JP)
(72) Inventor: MASUDA, Keiki, Osaka-shi Osaka 554-0022 (JP); IGUCHI, Haruhisa, Osaka-shi Osaka 554-0022 (JP); GOTO, Masashi, Osaka-shi Osaka 554-0022 (JP); TORIGOE, Toshihiko, Sapporo-shi Hokkaido 060-8556 (JP); HIROHASHI, Yoshihiko, Sapporo-shi Hokkaido 060-8556 (JP); MORITA, Rena, Sapporo-shi Hokkaido 060-8556 (JP)
(74) Representative: Held, Stephan
(86) International application number: PCT/JP2014/068595
(87) International publication number: WO 2015/005479

(57) **Abstract**

Provided is a tumor antigen peptide and the like that is useful in the prevention and/or treatment of cancer. The present invention pertains to: a peptide comprising the amino acid sequence of sequence number 3, sequence number 4, sequence number 5, sequence number 6, or sequence number 11; a polyepitope peptide resulting from joining a plurality of epitope peptides and containing at least one of the aforementioned peptides as one epitope peptide; a polynucleotide coding for at least one of the polyepitope peptides or the peptides; a pharmaceutical composition containing the above as active ingredients; a cancer prevention and/or treatment agent characterized by inducing CTLs; and the like.

## Description

### [Technical Field]

The present invention relates to an OR7C1-derived tumor antigen peptide, etc., which is useful as an agent for the prevention and/or treatment of a cancer.

### [Background Art]

In the elimination of tumor cells, virus-infected cells, etc. in a living body, cell-mediated immunity, in particular involving cytotoxic T cells (called CTLs), has an important function. In the case of the elimination of tumor cells, a CTL recognizes a complex between an antigen peptide (tumor antigen peptide) and an MHC (Major Histocompatibility Complex) class I antigen (called an HLA class I antigen in the case of humans) on a tumor cell and attacks and destroys the tumor cell. That is, a tumor antigen peptide is produced by intracellular degradation by a protease of a tumor-specific protein, that is, a tumor antigen protein, after it has been synthesized in the cell. The tumor antigen peptide thus produced binds to an MHC class I antigen (HLA class I antigen) in the endoplasmic reticulum to form a complex, which is transported to the cell surface, and is presented as an antigen. A tumor-specific CTL recognizes the complex involved in this antigen presentation, and an anti-tumor effect is exhibited via cytotoxic action, lymphokine production, etc. Accompanying the elucidation of such a series of actions, a therapy in which a tumor antigen protein or a tumor antigen peptide is utilized as a so-called cancer immunotherapy agent (cancer vaccine) to thus enhance cancer-specific CTLs in the body of a cancer patient is in the process of being developed.

OR7C1 (olfactory receptor family 7, subfamily C, member 1 (Olfactory receptor, family 7, subfamily C, member 1)) is a G protein-coupled receptor (G-protein-coupled receptors; GPCR) belonging to the olfactory receptor family. It has been clarified that OR7C1 is a gene that is expressed in SP (Side Population) cells of SW480 cells, HCT15 cells, and HT29 cells, which are human colon cancer cell lines, and LHK2 cells, which is a human lung cancer cell line (Patent Document 1). SP cells are considered to be a fraction that is rich in cancer stem cells, and have been reported to have high tumorigenicity.

Cancer stem cells are thought to be the main cause for the recurrence or metastasis of a cancer, and it has been pointed out in recent years that in the treatment of a cancer it is important to target cancer stem cells and suppress their proliferation and migration. In fact, when SW480 cells are transfected with an siRNA against OR7C1, the SP cell fraction proportion and tumorigenicity are greatly reduced, and it is therefore thought that OR7C1 plays an essential role in the function of cancer stem cells.

Furthermore, since OR7C1 is expressed only in the testis in normal tissue, it is classified as a 'cancer-testis antigen'. It is widely recognized that the cancer-testis antigen is a promising target molecule for a cancer vaccine.

Moreover, OR7C1 is a promising target molecule for a cancer vaccine, and in fact an OR7C1-derived partial peptide has been reported as an HLA-A24-binding tumor antigen peptide (Patent Document 1).

As described above, a CTL involved in tumor immunity recognizes a complex between a tumor antigen peptide and an HLA class I antigen on a tumor cell and attacks and destroys the tumor cell. In general, HLA-A02 restriction and HLA-A24 restriction of the tumor antigen peptide are independent of each other, and it is known that HLA-A02 and HLA-A24, which are found in Western people and Japanese people, exhibit different antigen specificity. With regard to OR7C1, an OR7C1-derived tumor antigen peptide that can bind to HLA-A24 is known (Patent Document 1), but an OR7C1-derived tumor antigen peptide that can bind to HLA-A02 and one that can bind to both HLA-A02 and HLA-A24 have not yet been found.

### [Prior Art Documents]

### [Patent Documents]

[Patent Document 1] International Patent Application WO No. 2010/050190

### [Summary of the Invention]

### [Problems to be Solved by the Invention]

It is an object of the present invention to provide an OR7C1-derived tumor antigen peptide that can bind to both HLA-A02 and HLA-A24 and a pharmaceutical composition, etc. useful for the prevention and/or treatment of a cancer containing the above as an active ingredient.

### [Means for Solving the Problems]

As a result of an intensive investigation by the present inventors, a tumor antigen peptide, that is a partial peptide of OR7C1 (Olfactory receptor, family 7, subfamily C, member 1) registered as Genbank Accession No. NP_945182 (SEQ ID No: 2) and that can bind to both HLA-A02 and HLA-A24 has been found.

Furthermore, the present inventors have also found that said tumor antigen peptide can be used as an inducer for a cancer-specific CTL in vivo or in vitro, that is, as a cancer vaccine. Said tumor antigen peptide is useful as an agent for the prevention and/or treatment of a cancer (tumor) disease such as colon cancer, lung cancer, or ovarian cancer. Moreover, said tumor antigen peptide is also useful as a tumor marker for a cancer (tumor) such as colon cancer, lung cancer, or ovarian cancer.

That is, the present invention is as listed below:
[1] A peptide consisting of an amino acid sequence represented by any of SEQ ID No: 3, SEQ ID No: 4, SEQ ID No: 5, SEQ ID No: 6, and SEQ ID No: 11;
[2] a peptide consisting of an amino acid sequence, the amino acid sequence being
   (1) an amino acid sequence having an amino acid at the C terminal replaced by a hydrophobic amino acid, and/or
   (2) an amino acid sequence having one to several amino acids added to the N terminal and/or the C terminal,
   in any of SEQ ID No: 3, SEQ ID No: 4, SEQ ID No: 5, SEQ ID No: 6, and SEQ ID No: 11;
[3] the peptide according to [2], wherein an amino acid at the C terminal of the amino acid sequence represented by any of SEQ ID No: 3, SEQ ID No: 4, SEQ ID No: 5, SEQ ID No: 6, and SEQ ID No: 11 is replaced by leucine, valine, or isoleucine;
[4] the peptide according to [2], wherein one amino acid is added to the N terminal or the C terminal of the amino acid sequence represented by any of SEQ ID No: 3, SEQ ID No: 4, SEQ ID No: 5, SEQ ID No: 6, and SEQ ID No: 11;
[5] a polyepitope peptide which comprises a plurality of epitope peptides linked together, wherein at least one of said epitope peptides is the peptide according to any one of [1] to [4];
[6] a pharmaceutical composition comprising as an active ingredient the peptide according to any one of [1] to [4] or the polyepitope peptide according to [5];
[7] the pharmaceutical composition according to [6], wherein the pharmaceutical composition further comprises an adjuvant;
[8] a vaccine for the prevention and/or treatment of a cancer, the vaccine comprising as an active ingredient the peptide according to any one of [1] to [4] or the polyepitope peptide according to [5];
[9] an agent for the prevention and/or treatment of a cancer, the agent comprising as an active ingredient the peptide according to any one of [1] to [4] or the polyepitope peptide according to [5];
[10] an agent for inducing a cytotoxic T cell (CTL), the agent comprising as an active ingredient the peptide according to any one of [1] to [4] or the polyepitope peptide according to [5];
[11] a polynucleotide encoding at least one of the peptide according to any one of [1] to [4] or the polyepitope peptide according to [5];
[12] an expression vector comprising the polynucleotide according to [11];
[13] a composition for gene transfer, the composition comprising the expression vector according to [12];
[14] a pharmaceutical composition for the treatment or prevention of a cancer, comprising as an active ingredient either (a) or (b) below:
   (a) the polynucleotide according to [11]
   (b) the expression vector according to [12];
[15] a method for producing an antigen-presenting cell, comprising contacting in vitro a cell having an antigen-presenting ability with (a) or (b) below:
   (a) the peptide according to any one of [1] to [4] or the polyepitope peptide according to [5],
   (b) a polynucleotide encoding at least one of the peptides described in (a) above;
[16] a method for inducing a CTL, comprising contacting in vitro a peripheral blood lymphocyte with either (a) or (b) below:
   (a) the peptide according to any one of [1] to [4] or the polyepitope peptide according to [5],
   (b) a polynucleotide encoding at least one of the peptides described in (a) above;
[17] an HLA multimer comprising an HLA and the peptide according to any one of [1] to [4];
[18] a diagnostic agent comprising the HLA multimer according to [17];
[19] a TCR-like antibody that recognizes a complex between an HLA and the peptide according to any one of [1] to [4];
[20] a tumor-detecting agent comprising the TCR-like antibody according to [19]; and
[21] a diagnostic agent for screening a patient to whom the pharmaceutical composition according to [6], [7], or [14] can be applied effectively, the diagnostic agent comprising the HLA multimer according to [17] or the TCR-like antibody according to [19].

### [Effects of the Invention]

In accordance with the present invention, a tumor antigen peptide that is useful as a CTL inducer and a pharmaceutical composition, etc., comprising the above as an active ingredient, that is useful for the prevention and/or treatment of a cancer are provided.

### [Brief Description of Drawings]

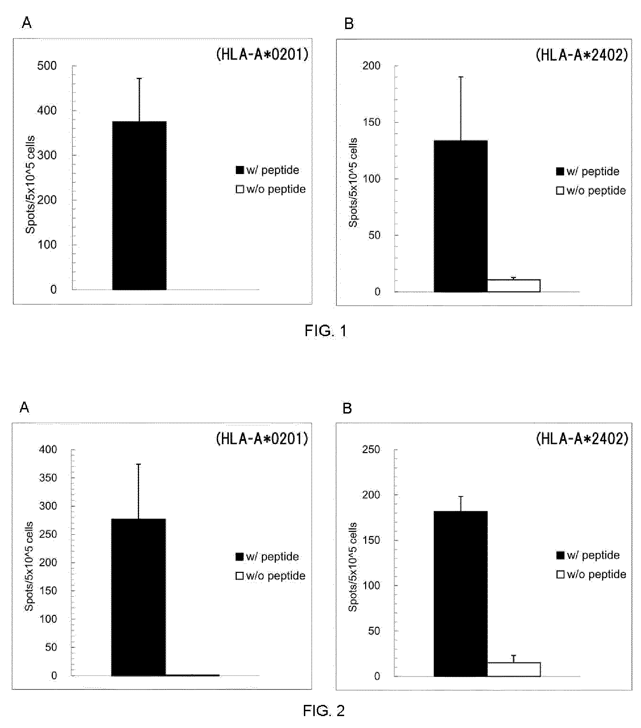

[FIG. 1] A diagram showing the results of evaluation of in vitro CTL inducibility of the peptide represented by SEQ ID No: 3 by means of an interferonγ ELISPOT assay. The ordinate denotes the number of spots per 500, 000 seeded cells. A denotes the result of a test using an HLA-A*02:01 transgenic mouse, and B denotes the result of a test using an HLA-A*24:02 transgenic mouse. The black bar ('w/ Peptide') and the white bar ('w/o Peptide') show the results of restimulation culturing of peptide-treated mouse-derived spleen cells in the presence or absence of the administered peptide respectively. That is, the differences in the figures between the black bar and the white bar denote the number of peptide-specific CTLs induced in the mouse living body by administration of each of the peptides.
[FIG. 2] A diagram showing the results of evaluation of in vitro CTL inducibility of the peptide represented by SEQ ID No: 4 by means of an interferonγ ELISPOT assay. The ordinate denotes the number of spots per 500, 000 seeded cells. A denotes the result of a test using an HLA-A*02:01 transgenic mouse, and B denotes the result of a test using an HLA-A*24:02 transgenic mouse. The black bar ('w/Peptide') and the white bar ('w/o Peptide') show the results of restimulation culturing of peptide-treated mouse-derived spleen cells in the presence or absence of the administered peptide respectively. That is, the differences in the figures between the black bar and the white bar denote the number of peptide-specific CTLs induced in the mouse living body by administration of each of the peptides.

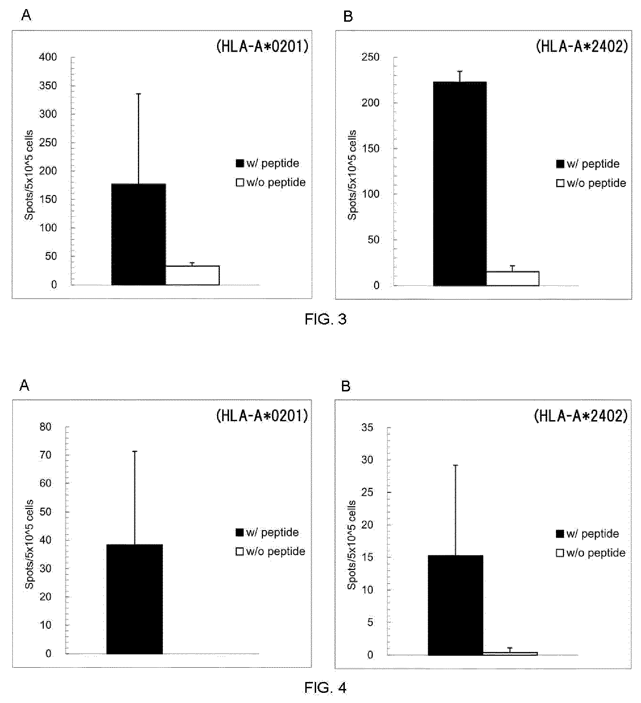

[FIG. 3] A diagram showing the results of evaluation of in vitro CTL inducibility of the peptide represented by SEQ ID No: 5 by means of an interferonγ ELISPOT assay. The ordinate denotes the number of spots per 500, 000 seeded cells. A denotes the result of a test using an HLA-A*02:01 transgenic mouse, and B denotes the result of a test using an HLA-A*24:02 transgenic mouse. The black bar ('w/Peptide') and the white bar ('w/o Peptide') show the results of restimulation culturing of peptide-treated mouse-derived spleen cells in the presence or absence of the administered peptide respectively. That is, the differences in the figures between the black bar and the white bar denote the number of peptide-specific CTLs induced in the mouse living body by administration of each of the peptides.
[FIG. 4] A diagram showing the results of evaluation of in vitro CTL inducibility of the peptide represented by SEQ ID No: 6 by means of an interferonγ ELISPOT assay. The ordinate denotes the number of spots per 500, 000 seeded cells. A denotes the result of a test using an HLA-A*02:01 transgenic mouse, and B denotes the result of a test using an HLA-A*24:02 transgenic mouse. The black bar ('w/Peptide') and the white bar ('w/o Peptide') show the results of restimulation culturing of peptide-treated mouse-derived spleen cells in the presence or absence of the administered peptide respectively. That is, the differences in the figures between the black bar and the white bar denote the number of peptide-specific CTLs induced in the mouse living body by administration of each of the peptides.

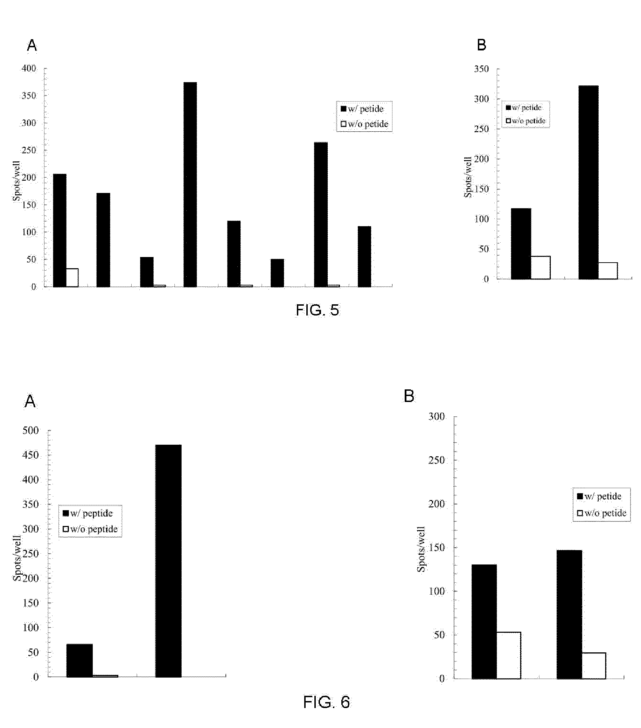

[FIG. 5] A diagram showing the results of evaluation of CTL inducibility of the peptide represented by SEQ ID No: 3 using human peripheral blood mononuclear cells. A denotes the result of a test using HLA-A*02:01-positive healthy individual-derived peripheral blood mononuclear cells, and B denotes the result of a test using HLA-A*24:02-positive healthy individual-derived peripheral blood mononuclear cells. The black bar and the white bar denote the results of restimulation and non-restimulation. That is, the differences in the figures between the black bar and the white bar denote the number of peptide-specific CTLs induced by stimulation culturing of the peptide. The ordinate denotes the number of spots observed in each well, and the abscissa denotes each of the positive wells. Furthermore, the black bar denotes the number of spots detected under peptide stimulation conditions, and the white bar denotes the number of spots detected under peptide non-pulse conditions (control).
[FIG. 6] A diagram showing the results of evaluation of CTL inducibility of the peptide represented by SEQ ID No: 5 using human peripheral blood mononuclear cells. A denotes the result of a test using HLA-A*02:01-positive healthy individual-derived peripheral blood mononuclear cells, and B denotes the result of a test using HLA-A*24:02-positive healthy individual-derived peripheral blood mononuclear cells. The black bar and the white bar denote the results of restimulation and non-restimulation. That is, the differences in the figures between the black bar and the white bar denote the number of peptide-specific CTLs induced by stimulation culturing of the peptide. The ordinate denotes the number of spots observed in each well, and the abscissa denotes each of the positive wells. Furthermore, the black bar denotes the number of spots detected under peptide stimulation conditions, and the white bar denotes the number of spots detected under peptide non-pulse conditions (control).

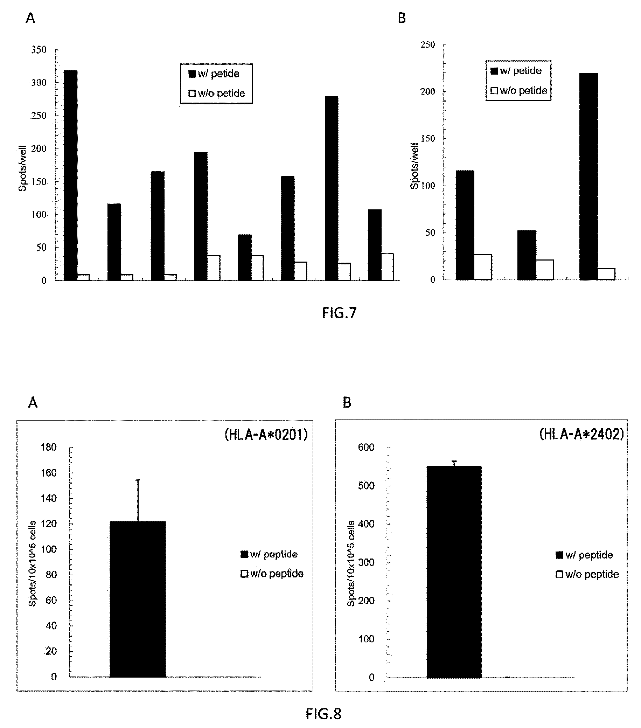

[FIG. 7] A diagram showing the results of evaluation of CTL inducibility of the peptide represented by SEQ ID No: 6 using human peripheral blood mononuclear cells. A denotes the result of a test using HLA-A*02:01-positive healthy individual-derived peripheral blood mononuclear cells, and B denotes the result of a test using HLA-A*24:02-positive healthy individual-derived peripheral blood mononuclear cells. The black bar and the white bar denote the results of restimulation and non-restimulation. That is, the differences in the figures between the black bar and the white bar denote the number of peptide-specific CTLs induced by stimulation culturing of the peptide. The ordinate denotes the number of spots observed in each well, and the abscissa denotes each of the positive wells. Furthermore, the black bar denotes the number of spots detected under peptide stimulation conditions, and the white bar denotes the number of spots detected under peptide non-pulse conditions (control).
[FIG. 8] A diagram showing the results of evaluation of in vitro CTL inducibility of the peptide represented by SEQ ID No: 11 by means of an interferonγ ELISPOT assay. The ordinate denotes the number of spots per 500,000 seeded cells. A denotes the result of a test using an HLA-A*02:01 transgenic mouse, and B denotes the result of a test using an HLA-A*24:02 transgenic mouse. Furthermore, the black bar ('w/Peptide') and the white bar ('w/o Peptide') show the results of restimulation culturing of peptide-treated mouse-derived spleen cells in the presence or absence of the administered peptide respectively. That is, the differences in the figures between the black bar and the white bar denote the number of peptide-specific CTLs induced in the mouse living body by administration of each of the peptides.

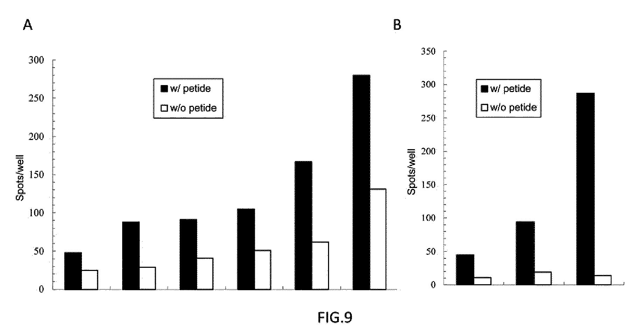

[FIG. 9] A diagram showing the results of evaluation of CTL inducibility of the peptide represented by SEQ ID No: 11 using human peripheral blood mononuclear cells. A denotes the result of a test using HLA-A*02:01-positive healthy individual-derived peripheral blood mononuclear cells, and B denotes the result of a test using HLA-A*24:02-positive healthy individual-derived peripheral blood mononuclear cells. The black bar and the white bar denote the results of restimulation and non-restimulation. That is, the differences in the figures between the black bar and the white bar denote the number of peptide-specific CTLs induced by stimulation culturing of the peptide. The ordinate denotes the number of spots observed in each well, and the abscissa denotes each of the positive wells. Furthermore, the black bar denotes the number of spots detected under peptide stimulation conditions, and the white bar denotes the number of spots detected under peptide non-pulse conditions (control).

### [Modes for Carrying Out the Invention]

### (1) Peptide of the present invention

The 'epitope peptide' referred to in the present invention means a peptide that binds to an MHC (an HLA for humans) and is subjected to antigen presentation on the cell surface. The epitope peptide includes a CTL epitope peptide that binds to an MHC class I, is subjected to antigen presentation, and is recognized by a CD8-positive T cell, and a helper epitope peptide that binds to an MHC class II, is subjected to antigen presentation, and is recognized by a CD4-positive T cell.

Among epitope peptides, a protein-derived peptide that is specifically or over expressed in a tumor cell is in particular called a tumor antigen peptide. The antigen presentation referred to a phenomenon in which a peptide present within a cell binds to an MHC and this MHC/antigen peptide complex is localized on the cell surface. As described above, it is known that an antigen presented on a cell surface is recognized by a T cell, etc. and then activates cell-mediated immunity or humoral immunity; since an antigen presented by an MHC class I activates cell-mediated immunity and is also recognized by a T cell receptor of a naive T cell to thus induce the naive T cell to become a CTL having cytotoxic activity, a tumor antigen peptide used in immunotherapy is preferably an antigen-presenting peptide that binds to an MHC class I.

In the present invention, a 'tumor' includes a benign tumor and a malignant tumor (cancer, malignant neoplasm). A cancer includes a hematopoietic tumor, an epithelial malignant tumor (carcinoma), and a nonepithelial malignant tumor (sarcoma).

In one embodiment, the peptide of the present invention is a human OR7C1 partial peptide described in SEQ ID No: 2, the peptide binding to an MHC, and in particular to an HLA; it is preferably a peptide that is subjected to antigen presentation by means of an MHC, in particular an HLA, and more preferably a peptide that is subjected to antigen presentation by means of an MHC, in particular an HLA, and can induce a CTL. There are several types of HLA; the peptide of the present invention preferably can bind to an HLA class I, more preferably can bind to HLA-A02, and yet more preferably can bind to both HLA-A02 and HLA-A24. The peptide of the present invention may be subjected to a treatment such as processing prior to binding to an MHC, and a peptide that forms an epitope peptide as a result of such a treatment is also included in the peptide of the present invention. Therefore, the amino acid length of the peptide of the present invention is not particularly limited as long as it is a sequence containing an amino acid sequence of an epitope peptide. However, it is preferable that the peptide of the present invention itself is an epitope peptide, and therefore the amino acid length is preferably on the order of about 9 to about 11 amino acids.

In a preferred embodiment, the peptide of the present invention includes a peptide comprising the same amino acid sequence as the amino acid sequence described in SEQ ID No: 3, SEQ ID No: 4, SEQ ID No: 5, or SEQ ID No: 6, and it is more preferable that all of these amino acid sequences are partial peptides of the OR7C1 described above. More preferably, it is a peptide consisting of the same amino acid sequence as the amino acid sequence described in SEQ ID No: 3, SEQ ID No: 4, SEQ ID No: 5, or SEQ ID No: 6. The peptides represented by SEQ ID Nos: 3 to 6 are peptides consisting of the 10 amino acids of positions 117 to 126, the 10 amino acids of positions 276 to 285, the 9 amino acids of positions 118 to 126, and the 11 amino acids of positions 34 to 44 of the above OR7C1, respectively, and it has been found by the present inventors that all of the peptides can bind to both HLA-A02 and HLA-A24.

The peptide of the present invention may have its N terminal and/or C terminal modified. Specific examples of the modification include N-alkanoylation (for example, acetylation), N-alkylation (for example, methylation), a C-terminal alkyl ester (for example, an ethyl ester), and a C-terminal amide (for example a carboxamide) .

In another embodiment of the present invention, the peptide of the present invention includes a peptide comprising an amino acid sequence that is an OR7C1 partial peptide that binds to an MHC, in particular an HLA, the amino acid sequence having one to several amino acids added to the N terminal and/or the C terminal. A particularly preferred embodiment is a peptide consisting of an amino acid sequence represented by SEQ ID No: 3, SEQ ID No: 4, SEQ ID No: 5, or SEQ ID No: 6, the amino acid sequence having one to several amino acids added to the N terminal and/or the C terminal. The 'one to several amino acids' that are added to the N terminal and/or the C terminal specifically means 1 to 5 amino acids; examples include 1, 2, 3, 4, or 5 amino acids, and 1 is particularly preferable.

Furthermore, in another embodiment of the present invention, the peptide of the present invention includes a peptide comprising an amino acid sequence that is an OR7C1 partial peptide that binds to an MHC, in particular an HLA, the amino acid sequence having its C terminal amino acid, N terminal amino acid, and/or second amino acid, and preferably C terminal amino acid, replaced by a hydrophobic amino acid. A particularly preferred embodiment is a peptide consisting of an amino acid sequence represented by SEQ ID No: 3, SEQ ID No: 4, SEQ ID No: 5, or SEQ ID No: 6, the amino acid sequence having its C terminal amino acid and/or second amino acid, and preferably C terminal amino acid, replaced by a hydrophobic amino acid. In the present invention, a 'hydrophobic amino acid' means an amino acid that does not have intramolecular polarity, and among others it preferably means glycine, alanine, valine, leucine, isoleucine, methionine, proline, phenylalanine, and tryptophan, which are natural amino acids. In the present invention, among these hydrophobic amino acids, leucine, valine, and isoleucine are particularly preferable. Therefore, a preferred peptide in the embodiment includes one in which the C terminal amino acid is replaced by leucine, valine, or isoleucine, for example, a peptide (SEQ ID No: 11) in which the C terminal valine of the peptide of SEQ ID No: 3 is replaced by isoleucine. In addition, the amino acid sequence of SEQ ID No: 11 coincides with the amino acid sequence of a partial peptide of amino acid positions 117 to 126 in a polypeptide encoded by a single nucleotide polymorphism variant nucleotide sequence in which the position 376 guanine (G) in the base sequence of human OR7C1 gene (Genbank Accession No. NM_198944, SEQ ID No: 1) is replaced by adenine (A) (that is, a variant in which the position 126 valine (V) in the amino acid sequence represented by SEQ ID No: 2 is replaced by isoleucine (I)).

A person skilled in the art will understand that modification to add one to several amino acids to the N terminal and/or C terminal and modification to replace a C terminal amino acid, N terminal amino acid, and/or second amino acid with a hydrophobic amino acid can be combined. Therefore, the peptide of the present invention includes a peptide comprising an amino acid sequence that is an OR7C1 partial peptide that binds to an MHC, in particular an HLA, one to several amino acids being added to the N terminal and/or C terminal of the peptide comprising the amino acid sequence having its C terminal amino acid, N terminal amino acid, and/or second amino acid, and preferably C terminal amino acid, replaced by a hydrophobic amino acid. Among them, a peptide in which 1, 2, 3, 4, or 5 amino acids, and particularly preferably 1 amino acid, is added to the N terminal and/or C terminal of the peptide represented by SEQ ID No: 11 is preferable.

Synthesis of the peptide of the present invention may be carried out in accordance with a known method used in usual peptide chemistry. Such a known method includes methods described in the literature (Peptide Synthesis, Interscience, New York, 1966; The Proteins, Vol. 2, Academic Press Inc., New York, 1976; Peptide Synthesis, Maruzen Co., Ltd., 1975; Basics and Experiments of Peptide Synthesis, Maruzen Co., Ltd., 1985; Development of Pharmaceuticals Seq. Vol. 14 Peptide Synthesis, Hirokawa Shoten Co., 1991, these publications forming part of the present application by reference), etc.

With regard to the peptide of the present invention, in vivo activity can be confirmed by subjecting it to a CTL induction method, which is described later, an assay using an animal model for human (WO02/47474, Int J. Cancer: 100, 565-570 (2002)), etc.

The peptide of the present invention further includes a peptide in which a plurality of epitope peptides containing at least one of the peptides of the present invention are linked (polyepitope peptide). Therefore, specific examples of the peptide of the present invention include a peptide that is the above polyepitope peptide and has CTL-inducing activity.

The polyepitope peptide of the present invention may specifically be defined as
(i) a peptide in which the peptide of the present invention (epitope peptide) and any one or more CTL epitope peptides other than the peptide of the present invention are linked directly or via a spacer as appropriate,
(ii) a peptide in which the peptide of the present invention and any one or more helper epitope peptides are linked directly or via a spacer as appropriate, or
(iii) a peptide in which a polyepitope peptide described in (i) above and further one or more helper epitope peptides are linked directly or via a spacer as appropriate, the peptide being subjected to processing within an antigen-presenting cell, and the epitope peptide thus formed being presented on the antigen-presenting cell, thus leading to CTL-inducing activity.

Here, the CTL epitope peptide other than the peptide of the present invention in (i) is not particularly limited, and specific examples include an OR7C1-derived peptide (for example, a peptide described in International Patent Application No. WO2010/050190).

The spacer is not particularly limited as long as it does not adversely affect processing within an antigen-presenting cell, and is preferably a linker that is linked to each epitope peptide via a peptide bond, examples including a peptide linker in which several amino acids are linked and a linker having an amino group and a carboxyl group at each end. Specific examples include a glycine linker or a PEG (polyethylene glycol) linker; examples of the glycine linker include polyglycine (for example a peptide comprising six glycines; Cancer Sci, Vol. 103, p. 150-153), and examples of the PEG linker include a linker derived from a compound having an amino group and a carboxy group at each end (for example, H₂N-(CH₂)₂-(OCH₂CH₂)₃-COOH; Angew. Chem. Int. Ed. 2008, 47, 7551-7556).

With regard to the epitope peptide of the present invention contained in the polyepitope peptide of the present invention, one or more types may be selected. That is, a plurality of identical epitope peptides may be linked, or a plurality of different epitope peptides may be linked. Naturally, even when two or more types of epitope peptides are selected, a plurality of one or more types of selected epitope peptides may be linked. Similarly, with regard to the epitope peptide other than the peptide of the present invention, a plurality of types and/or a plurality of epitope peptides may be linked. The polyepitope peptide of the present invention may be one in which 2 to 12 epitope peptides are linked, is preferably one in which 2, 3, 4, 5, 6, 7, 8, 9, 10, 11, or 12 epitope peptides are linked, and is most preferably one in which 2 epitope peptides are linked.

When the epitope peptide that is linked to the peptide of the present invention is a helper epitope peptide, examples of the helper epitope peptide used include hepatitis B virus-derived HBVc128-140 and tetanus toxin-derived TT947-967. The length of the helper epitope peptide is for example on the order of 13 to 30 amino acids, and preferably on the order of 13 to 17 amino acids.

Such a peptide in which a plurality of epitope peptides are linked (polyepitope peptide) may also be produced by a standard peptide synthesis method as described above. Furthermore, based on information regarding the sequence of a polynucleotide encoding such a polyepitope peptide in which a plurality of epitope peptides are linked, it may be produced using standard DNA synthesis and genetic engineering methods.

That is, said polynucleotide is inserted into a known expression vector, a host cell is transformed by means of the recombinant expression vector thus obtained to give a transformant, the transformant is cultured, and the target polyepitope peptide in which a plurality of epitopes are linked can be produced by recovery from the culture. These methods may be carried out in accordance with methods described in the literature as described above (Molecular Cloning, T. Maniatis et al., CSH Laboratory (1983), DNA Cloning, D M. Glover, IRL PRESS (1985)).

The polyepitope peptide thus produced in which a plurality of epitope peptides are linked is subjected to the above in vitro assay or an in vivo assay using an animal model for human described in WO02/47474 and Int J. Cancer: 100, 565-570 (2002) (these publications forming part of the present application by reference), etc., thus enabling CTL-inducing activity to be confirmed.

The peptide of the present invention (including the polyepitope peptide) is useful for the prevention and/or treatment of a cancer, etc. as described in the present specification, and may be an active ingredient of a pharmaceutical composition. Furthermore, the peptide of the present invention may be for the prevention and/or treatment of a cancer. Moreover, the present invention also relates to use of the peptide of the present invention in the production of a medicament for the prevention and/or treatment of a cancer.

### (2) Polynucleotide of the present invention

The polynucleotide of the present invention includes a polynucleotide that encodes at least one of the peptides of the present invention. The polynucleotide of the present invention may be any of cDNA, mRNA, cRNA, or synthetic DNA. It may have either a single-strand or a double-strand configuration. Specific examples include a polynucleotide consisting of a nucleotide sequence encoding an amino acid sequence described in SEQ ID No: 3, SEQ ID No: 4, SEQ ID No: 5, SEQ ID No: 6 or SEQ ID No: 11, and a polynucleotide consisting of a nucleotide sequence encoding so that it can express a polyepitope peptide in which any two or more peptides selected from SEQ ID Nos: 3, 4, 5, 6, and 11 are linked or a peptide selected from SEQ ID Nos: 3, 4, 5, 6, and 11 and a helper epitope are linked.

The polynucleotide of the present invention may take on either a single strand or a double strand configuration. When the polynucleotide of the present invention is a double strand, a recombinant expression vector expressing the peptide of the present invention may be produced by inserting the polynucleotide of the present invention into an expression vector. That is, the scope of the polynucleotide of the present invention includes a recombinant expression vector produced by inserting the double strand polynucleotide of the present invention into an expression vector.

The polynucleotide of the present invention is useful for the prevention and/or treatment of a cancer, etc. as described in the present specification, and may be an active ingredient of a pharmaceutical composition. Furthermore, the polynucleotide of the present invention may be for the prevention and/or treatment of a cancer. Moreover, the present invention also relates to use of the polynucleotide of the present invention in the production of a medicament for the prevention and/or treatment of a cancer.

With regard to an expression vector used in the present invention, various types may be used according to the host used, the intended application, etc., and a person skilled in the art may select it as appropriate. Examples of expression vectors that can be used in the present invention include a plasmid, a phage vector, and a virus vector. For example, when the host is Escherichia coli, examples of the vector include plasmid vectors such as pUC118, pUC119, pBR322, and pCR3 and phage vectors such as λZAPII and λgt11. When the host is a yeast, examples of the vector include pYES2 and pYEUra3. When the host is an insect cell, examples include pAcSGHisNT-A. When the host is an animal cell, examples include plasmid vectors such as pCEP4, pKCR, pCDM8, pGL2, pcDNA3.1, pRc/RSV, and pRc/CMV and virus vectors such as a retrovirus vector, an adenovirus vector, and an adeno-associated virus vector.

The vector may have as appropriate a factor such as a promoter capable of inducing expression, a gene encoding a signal sequence, a selection marker gene, or a terminator. Furthermore, in order to make isolation and purification easy, a sequence for expression as a fusion protein with thioredoxin, a His tag, GST (glutathione S-transferase), etc. may be added. In this case, a GST fusion protein vector (pGEX4T, etc.) having an appropriate promoter (lac, tac, trc, trp, CMV, SV40 early promoter, etc.) that functions within a host cell, a vector having a tag sequence such as Myc or His (pcDNA3.1/Myc-His, etc.) and, furthermore, a vector expressing a fusion protein with thioredoxin and a His tag (pET32a), etc. may be used.

Transforming a host with the expression vector prepared as above enables a transformed cell containing the expression vector to be prepared. The host used here may be any cell as long as the function of the polypeptide of the present invention is not impaired, and examples include an Escherichia coli, a yeast, an insect cell, and an animal cell. Examples of the Escherichia coli include E.coli K-12 strain HB101, C600, JM109, DHSα, and AD494 (DE3). Examples of the yeast include Saccharomyces cerevisiae. Examples of the animal cell include L929 cells, BALB/c3T3 cells, C127 cells, CHO cells, COS cells, Vero cells, Hela cells, and 293-EBNA cells. Examples of the insect cell include sf9.

As a method for introducing an expression vector into a host cell, a standard introduction method suitable for the host cell may be used. Specific examples include a calcium phosphate method, a DEAE-dextran method, an electroporation method, and a method using a lipid for gene transfer (Lipofectamine, Lipofectin; Gibco-BRL). After introduction, culturing is carried out in a standard medium containing a selection marker, thus enabling a transformed cell in which the expression vector has been introduced into the host cell to be selected.

Continuing culturing the transformed cell thus obtained under suitable conditions enables the peptide of the present invention to be produced. The peptide thus obtained may be further isolated and purified by usual biochemical purification means. Examples of purification means include salting out, ion-exchange chromatography, adsorption chromatography, affinity chromatography, and gel filtration chromatography. When the peptide of the present invention is expressed as a fusion protein with a thioredoxin, a His tag, a GST, etc. as described above, isolation and purification may be carried out by a purification method utilizing the properties of the fusion protein or the tag.

The polynucleotide encoding the peptide of the present invention may have a DNA configuration or an RNA configuration. These polynucleotides of the present invention may be easily produced by standard methods known in the present technical field based on amino acid sequence information of the peptide of the present invention and DNA sequence information encoded thereby. Specifically, it may be produced by standard DNA synthesis, amplification by means of PCR, etc.

The polynucleotide encoding the peptide of the present invention includes a polynucleotide encoding the epitope peptide.

### (3) CTL inducer comprising a peptide of the present invention as active ingredient,

The peptide of the present invention has CTL-inducing activity, and can be a CTL inducer as a tumor antigen peptide.

That is, peripheral blood lymphocytes are isolated from a person who is positive for an HLA-A02 antigen or an HLA-A24 antigen, they are stimulated in vitro by adding the peptide of the present invention, and CTLs that specifically recognize an HLA-A02 antigen-positive cell or an HLA-A24 antigen-positive cell that have been pulsed with the peptide can be induced (J. Immunol., 154, p. 2257, 1995). The presence or absence of CTL induction may be confirmed by measuring for example the amount of various cytokines (for example IFN-γ) produced by CTLs when reacting with an antigen peptide-presenting cell, by means of for example an ELISA method, etc. It may also be confirmed by a method for measuring CTL toxicity toward an antigen peptide-presenting cell labeled with ⁵¹Cr (⁵¹Cr release assay, Int. J. Cancer, 58: p317, 1994).

Furthermore, a CTL clone may be established by a method described in Int. J. Cancer, 39, 390-396, 1987, N. Eng. J. Med, 333, 1038-1044, 1995, etc.

A CTL induced by the peptide of the present invention has a cytotoxic action toward a cell presenting the peptide of the present invention as an antigen and the ability to produce a lymphokine. Since the peptide of the present invention is a tumor antigen peptide as described above, it can exhibit an anti-tumor action, and preferably an anti-cancer action, via the above functions. Therefore, the peptide of the present invention and a CTL induced thereby can be an active ingredient of a medicament or a pharmaceutical composition for the prevention and/or treatment of a cancer.

When a CTL inducer containing the peptide of the present invention as an active ingredient is administered to a cancer patient, the peptide of the present invention is presented to an HLA-A02 antigen or HLA-A24 antigen of an antigen-presenting cell, a CTL that is specific to a complex between the HLA-A02 antigen or HLA-A24 antigen and the presented peptide proliferates and destroys the cancer cells, and as a result, the cancer can be prevented and/or treated. Therefore, a CTL inducer containing the peptide of the present invention as an active ingredient can preferably be used for a subject who is positive for an HLA-A02 antigen or HLA-A24 antigen and who has an OR7C1-positive cancer. Examples of OR7C1-positive cancers include cancers (tumors) such as colon cancer, lung cancer, and ovarian cancer, and the CTL inducer of the present invention may be used for the prevention and/or treatment of such cancers.

The 'prevention' of a cancer includes not only preventing a patient from having a cancer but also prevention of recurrence in a patient who has been subjected to surgery to remove a primary tumor and prevention of metastasis of a tumor that could not be completely removed by a cancer treatment such as surgery, radiotherapy, drug therapy, etc. Furthermore, the 'treatment' of a cancer includes not only curing and improvement of the symptoms of a cancer that reduces the size of the cancer but also prevention of cancer cell proliferation or tumor enlargement, or suppression of metastasis of cancer cells from a primary focus.

A CTL inducer containing the peptide of the present invention as an active ingredient is particularly effective for an HLA-A02- or HLA-A24-positive cancer patient who has a cancer positive for OR7C1 described in SEQ ID No: 2. Specifically, it may be used for the prevention or treatment of a cancer (tumor) such as for example colon cancer, lung cancer, or ovarian cancer.

A CTL inducer containing the peptide of the present invention as an active ingredient may be one that contains a single CTL epitope (the peptide of the present invention) as an active ingredient or one that contains as an active ingredient a polyepitope peptide having another peptide (CTL epitope or helper epitope) linked thereto. In recent years, it has been shown that a polyepitope peptide having a plurality of linked CTL epitopes (antigen peptides) has activity in efficiently inducing CTLs in vivo. For example, Journal of Immunology 1998, 161: 3186-3194 (this publication forms part of the present application by reference) describes the induction in vivo of a CTL that is specific to each CTL epitope by means of an approximately 30mer polyepitope peptide in which cancer antigen protein PSA-derived HLA-A2, -A3, -A11, and -B53 restriction CTL epitopes (antigen peptides) are linked. It is also shown that a polyepitope peptide in which a CTL epitope and a helper epitope are linked efficiently induces a CTL. When administered in the configuration of such a polyepitope peptide, the polyepitope peptide is incorporated into an antigen-presenting cell, and after that, individual antigen peptides that have been formed by intracellular degradation bind to an HLA antigen to thus form a complex, this complex is presented on the antigen-presenting cell surface at high density, a CTL specific to this complex proliferates efficiently in the body, and cancer cells are destroyed. In this way, the treatment or prevention of a cancer is promoted.

A CTL inducer containing the peptide of the present invention as an active ingredient may be administered as a mixture with a pharmaceutically acceptable carrier, for example an appropriate adjuvant, or in combination therewith, so as to establish cell-mediated immunity effectively.

As the adjuvant, an adjuvant known in the present technical field such as one described in the literature (for example, Clin Infect Dis.: S266-70, 2000) may be applied, and specific examples include a gel type such as aluminum hydroxide, aluminum phosphate, or calcium phosphate, a bacterial type such as CpG, monophosphoryl lipid A (monophosphoryl lipid A; MPL), cholera toxin, Escherichia coli heat-labile toxin, pertussis toxin, or muramyl dipeptide (Muramyl dipeptide; MDP), an oil emulsion type (emulsion preparation) such as Freund's incomplete adjuvant, MF59, or SAF, a macromolecular nanoparticle type such as an immunostimulatory complex (Immunostimulatory complex; ISCOMs), a liposome, biodegradable microspheres (Biodegradable microsphere), or saponin-derived QS-21, a synthetic type such as a nonionic block copolymer, a muramyl peptide analog (Muramyl peptide analogue), a polyphosphazene, or a synthetic polynucleotide, and a cytokine type such as IFN-γ, IL-2, or IL-12.

Furthermore, the dosage form of a CTL inducer containing the peptide of the present invention as an active ingredient is not particularly limited, and examples include an oil emulsion (emulsion formulation), macromolecular nanoparticles, a liposome formulation, a particulate preparation bonded to beads having a diameter of a few µm, a lipid-bonded formulation, a microsphere formulation, and a microcapsule formulation.

Examples of an administration method include any known administration method such as intradermal administration, subcutaneous administration, intramuscular administration, or intravenous administration. The dose of the peptide of the present invention in a preparation may be adjusted as appropriate according to the target disease to be treated, the age and body weight of the patient, etc., but it is usually 0.0001 mg to 1000 mg, preferably 0.001 mg to 1000 mg, and more preferably 0.1 mg to 10 mg, this being preferably administered once in a few days to a few months.

As a method for making the peptide of the present invention actually act as a medicament, there is an in vivo method in which the peptide is directly introduced into the body as well as an ex vivo method in which a specific type of cells are collected from a person, the peptide of the present invention is made to act thereon in vitro, and the cells are returned into the body (Nikkei Science, April, 1994, pp. 20-45, Gekkan Yakuji, 36 (1), 23-48 (1994), Experimental Medicine Special Edition, 12 (15), (1994), references quoted therein, etc. , these publications forming part of the present application by reference), and a person skilled in the art can select a cell, an administration method, an administration configuration, and a dose appropriate for such a method.

### (4) CTL inducer containing the polynucleotide of the present invention as active ingredient

Since a cell in which the polynucleotide of the present invention is expressed becomes a cell that presents the peptide of the present invention as an antigen, it has the feature that it is recognized by a T cell via a T cell receptor. Therefore, the polynucleotide of the present invention can also become a CTL inducer. An induced CTL can exhibit, in the same way as for a CTL induced by the peptide of the present invention, an anti-tumor action via a cytotoxic action or the production of a lymphokine, and preferably an anti-cancer action. Therefore, the polynucleotide of the present invention can be an active ingredient of a medicament or a pharmaceutical composition for the treatment or prevention of a cancer. A CTL inducer containing the polynucleotide of the present invention as an active ingredient can treat and/or prevent a cancer by for example administering the polynucleotide of the present invention to a cancer patient and expressing them in the cancer patient.

For example, when the polynucleotide of the present invention incorporated into an expression vector is administered to a cancer patient by the method below, a tumor antigen peptide is highly expressed within antigen-presenting cells. The tumor antigen peptide thus produced subsequently binds to an HLA-A02 antigen or an HLA-A24 antigen to form a complex, this complex is presented at high density on the antigen-presenting cell surface, cancer-specific CTLs proliferate efficiently in the body, and the cancer cells are destroyed. As described above, the treatment or prevention of a cancer is achieved.

The CTL inducer containing the polynucleotide of the present invention as an active ingredient may preferably be used for an HLA-A02 antigen or HLA-A24 antigen-positive subject who has an OR7C1-positive cancer. Examples of the OR7C1-positive cancer include cancers (tumors) such as colon cancer, lung cancer, and ovarian cancer, and the CTL inducer of the present invention may be used for the prevention or treatment of these cancers.

As a method for administering the polynucleotide of the present invention and incorporating it into a cell, any method such as a method involving a virus vector and other methods (Nikkei Science, 1994, April, pp. 20-45, Gekkan Yakuji, 36 (1), 23-48 (1994), Experimental Medicine Special Edition, 12 (15), (1994), references quoted therein, etc. , these publications forming part of the present application by reference) may be employed.

Examples of a method involving a virus vector include a method in which the DNA of the present invention is integrated into for example a DNA virus or RNA virus such as a retrovirus, adenovirus, adeno-associated virus, herpes virus, vaccinia virus, poxvirus, poliovirus, or sindbis virus, and incorporation is carried out. Among them, a method involving a retrovirus, adenovirus, adeno-associated virus, vaccinia virus, etc. is particularly preferable.

Examples of other methods include a method in which an expression plasmid is directly administered intramuscularly (DNA vaccine method), a liposome method, a lipofectin method, a microinjection method, a calcium phosphate method, and an electroporation method, and a DNA vaccine method and a liposome method are particularly preferable.

In order to make the polynucleotide of the present invention actually act as a medicament, there are an in vivo method in which the polynucleotide is directly introduced into the body and an ex vivo method in which a specific type of cells are collected from a person, the polynucleotide of the present invention is incorporated into the cells in vitro, and the cells are returned into the body (Nikkei Science, 1994, April, pp. 20-45, Gekkan Yakuji, 36 (1), 23-48 (1994), Experimental Medicine Special Edition, 12 (15), (1994), references quoted therein, etc., these publications forming part of the present application by reference). An in vivo method is more preferable.

When the polynucleotide of the present invention is administered by an in vivo method, administration may be carried out by selecting as appropriate an administration route and an administration form according to the target disease to be treated, the symptoms, etc. For example, administration may be carried out in a form that can be injected into a vein, an artery, subcutaneously, intradermally, intramuscularly, etc. When administration is carried out by an in vivo method, for example, a formulation form such as a liquid may be employed, but it is usually made into an injection, etc. containing the polynucleotide of the present invention, which is an active ingredient, and a pharmaceutically acceptable carrier (carrier) may be added as necessary. With regard to a liposome or a membrane fusion liposome (Sendai virus (HVJ)-liposome, etc.) containing the polynucleotide of the present invention, a liposome preparation such as a suspension, a frozen agent, or a centrifugation-concentrated frozen agent may be employed.

The content of the polynucleotide of the present invention in a formulation may be adjusted as appropriate according to the target disease to be treated, the age and body weight of the patient, etc.; it is usually 0.0001 mg to 100 mg as a polynucleotide content, and preferably 0.001 mg to 10 mg of the polynucleotide of the present invention, it preferably being administered once in a few days to a few months.

A person skilled in the art can appropriately select a suitable cell, vector, administration method, administration form, and dose.

Furthermore, in recent years, it has been shown that a polynucleotide encoding a polyepitope peptide having a plurality of linked CTL epitopes (tumor antigen peptides) and a polynucleotide encoding a polyepitope peptide having a CTL epitope and a helper epitope that are linked have activity in efficiently inducing CTLs in vivo. For example, Journal of Immunology 1999, 162: 3915-3925 (this publication forms part of the present application by reference) reports that DNA encoding an epitope peptide (minigene) having six types of HBV-derived HLA-A2 restriction antigen peptides, three types of HLA-A11 restriction antigen peptides, and a helper epitope that are linked has induced CTLs for each epitope in vivo effectively.

Therefore, a CTL inducer active ingredient can be made by incorporating into an appropriate expression vector a polynucleotide prepared by linking one or more types of polynucleotide encoding the peptide of the present invention, and in some cases also linking a polynucleotide encoding another peptide. Such a CTL inducer can also employ the same administration method and administration form as described above.

### (5) Antigen-presenting cell of the present invention

The peptide or the polynucleotide of the present invention described above may be utilized in the treatment of a cancer patient, for example in vitro, as follows. That is, either of the peptide or the polynucleotide of the present invention and cells having antigen-presenting ability are brought into contact with each other in vitro, thus enabling antigen-presenting cells to be prepared. Specifically, the present invention provides an antigen-presenting cell presenting a complex between an HLA-A02 antigen or an HLA-A24 antigen and the peptide of the present invention on the cell surface of a cancer patient-derived isolated cell having antigen-presenting ability by bringing the cell into contact with either the peptide or the polynucleotide of the present invention in vitro, and a method for producing same.

The 'cell having antigen-presenting ability' is not particularly limited as long as it is a cell expressing on the cell surface an MHC, preferably an HLA-A02 antigen or an HLA-A24 antigen, that can present the peptide of the present invention, and among them it is preferably a professional antigen-presenting cell, and particularly preferably a dendritic cell, which is considered to have high antigen-presenting ability.

Furthermore, with regard to a substance that is added in order to prepare the antigen-presenting cell of the present invention from the cell having an antigen-presenting ability, it may be either the peptide or the polynucleotide of the present invention.

The antigen-presenting cell of the present invention is obtained by for example isolating cells having antigen-presenting ability from a cancer patient, and pulsing the cells with the peptide of the present invention in vitro so as to make them present a complex between an HLA-A02 antigen or an HLA-A24 antigen and the peptide of the present invention (Cancer Immunol. Immunother., 46: 82, 1998, J. Immunol., 158, p. 1796, 1997, Cancer Res., 59, p. 1184, 1999). When dendritic cells are used, for example, lymphocytes are separated from the peripheral blood of a cancer patient by the Ficoll method, non-adherent cells are then removed, adherent cells are cultured in the presence of GM-CSF and IL-4 to thus induce dendritic cells, and the dendritic cells are cultured and pulsed together with the peptide of the present invention, thus enabling the antigen-presenting cell of the present invention to be prepared.

Furthermore, when the antigen-presenting cell of the present invention is prepared by transfecting the cell having an antigen-presenting ability with the polynucleotide of the present invention, the polynucleotide may be in the form of a DNA or the form of an RNA. Specifically, in the case of a DNA, Cancer Res., 56: p. 5672, 1996 or J. Immunol., 161: p. 5607, 1998 (these publications forming part of the present application by reference) may be referred to, and in the case of an RNA, J. Exp. Med., 184: p. 465, 1996 (this publication forming part of the present application by reference) may be referred to.

The antigen-presenting cell can be an active ingredient of a CTL inducer. The CTL inducer containing the antigen-presenting cell as an active ingredient preferably contains physiological saline, phosphate buffered physiological saline (PBS), a medium, etc. in order to maintain the antigen-presenting cell stably. Examples of an administration method include intravenous administration, subcutaneous administration, and intradermal administration. Returning a CTL inducer containing such an antigen-presenting cell as an active ingredient to the body of the patient enables a cancer cell-specific CTL presenting the peptide of the present invention as an antigen to be efficiently induced in the body of a patient having an OR7C1-positive cancer, and as a result an antigen-presenting OR7C1-positive cancer that presents the peptide of the present invention as an antigen can be treated.

### (6) Cytotoxic T cell (CTL) of the present invention

The peptide and the polynucleotide of the present invention may be utilized in vivo in the treatment of a cancer patient as follows. That is, a CTL may be induced by bringing either the peptide or the polynucleotide of the present invention into contact with peripheral blood lymphocytes in vitro. Specifically, the present invention provides a CTL that is induced in vitro by bringing either the peptide or the polynucleotide of the present invention into contact with peripheral blood lymphocytes derived from a cancer patient, and a method for carrying out the induction.

In a melanoma for example, it has been confirmed that an adoptive immunotherapy in which a large number of intratumoral infiltrating T cells from the patient in question are cultured in vitro and returned to the patient has a therapeutic effect (J. Natl. Cancer. Inst., 86: 1159, 1994). Furthermore, in a mouse melanoma it has been confirmed that metastasis is suppressed by stimulating spleen cells in vitro with TRP-2 tumor antigen peptide so as to make CTLs specific to the tumor antigen peptide proliferate and administering the CTLs to a melanoma transplanted mouse (J. Exp. Med., 185: 453, 1997). This is based on the result that CTLs that specifically recognize a complex between a tumor antigen peptide and an MHC of an antigen-presenting cell proliferate in vitro. It is therefore considered that a therapy in which peripheral blood lymphocytes of a patient are stimulated in vitro using the peptide or the polynucleotide of the present invention to thus increase tumor-specific CTLs and the CTLs are subsequently returned to the patient will be useful.

The CTLs may be an active ingredient of a treatment agent or a preventive agent for a cancer. The treatment agent or the preventive agent preferably contains physiological saline, phosphate buffered physiological saline (PBS), a medium, etc. in order to maintain the CTLs stably. Examples of an administration method include intravenous administration, subcutaneous administration, and intradermal administration. Returning the cancer treatment or preventive agent containing such CTLs as an active ingredient to the body of a patient enables the cytotoxicity of the CTLs to cancer cells in the body of a patient having the OR7C1-positive cancer of the present invention to be promoted, and the cancer to be treated by destroying the cancer cells.

### (7) Tumor-specific CTL-detecting agent using the peptide of the present invention

The peptide of the present invention, in particular a peptide comprising an amino acid sequence described in SEQ ID No: 3, SEQ ID No: 4, SEQ ID No: 5, SEQ ID No: 6, or SEQ ID No: 11, is recognized by a tumor-specific CTL, and is therefore useful as a component of a tumor-specific CTL-detecting agent. Therefore, the present invention also relates to a tumor-specific CTL-detecting agent containing the peptide of the present invention. In one embodiment, the tumor-detecting agent and the tumor-specific CTL-detecting agent of the present invention contain an HLA multimer (monomer, dimer, tetramer, pentamer, or Dextramer) containing HLA-A02 or HLA-A24 and the peptide of the present invention.

For example, the HLA tetramer means a tetramer formed by biotinylating a complex (HLA monomer) in which the α chain and the β2 microglobulin of the HLA are associated with a peptide (antigen peptide) and binding it to avidin (Science 279: 2103-2106 (1998), Science 274: 94-96 (1996)). At present HLA tetramers containing various types of antigen peptides are commercially available (e.g. from Medical & Biological Laboratories Co., Ltd.), and an HLA tetramer containing the peptide of the present invention and HLA-A02 or HLA-A24 can be easily prepared. Furthermore, an HLA dimer and an HLA pentamer are also based on the same principle, the HLA monomer being formed into the dimer and the pentamer respectively. Therefore, an HLA multimer containing the peptide of the present invention and HLA-A02 or HLA-A24 is also one embodiment of the present invention.

Specific examples include an HLA tetramer containing a peptide consisting of an amino acid sequence described in SEQ ID No: 3, SEQ ID No: 4, SEQ ID No: 5, SEQ ID No: 6, or SEQ ID No: 11 and HLA-A02 or HLA-A24. The HLA tetramer is preferably fluorescently-labeled so that bound CTLs can be easily selected or detected by known detection means such as flow cytometry or a fluorescence microscope. Specific examples include HLA tetramers labeled with phycoerythrin (PE), fluorescein isothiocyanate (FITC), peridinin chlorophyll protein (PerCP), etc.

Examples of methods for producing an HLA tetramer include those described in the literature, such as Science 279: 2103-2106 (1998) and Science 274: 94-96 (1996), which are described in brief below.

First, Escherichia coli or mammalian cells that can express a protein are transfected with an HLA-A24 or HLA-A02 α chain expression vector and a β2 microglobulin expression vector and expression is carried out. Here, it is preferable to use Escherichia coli (for example, BL21). The monomer HLA-A24 or HLA-A02 complex thus obtained and the peptide of the present invention are mixed to thus form a soluble HLA-peptide complex. Subsequently, the C terminal site sequence of the α chain of HLA-A02 or HLA-A24 in the HLA-peptide complex is biotinylated with BirA enzyme. This biotinylated HLA-peptide complex and fluorescently-labeled avidin are mixed at a molar ratio of 4:1, thus preparing an HLA tetramer. In each of the above steps, it is preferable to carry out protein purification by means of gel filtration, etc.

### (8) Tumor-detecting agent targeted to the peptide of the present invention

The peptide of the present invention is presented by a cancer cell as a CTL epitope peptide and can therefore be used as a tumor marker. Because of this, a substance that can detect the peptide of the present invention as a tumor marker, for example, a TCR (T cell antigen receptor)-like antibody that recognizes a complex between the peptide of the present invention and HLA-A24 or HLA-A02 is useful as a tumor-detecting agent. Therefore, the present invention also relates to a tumor-detecting agent that includes a substance that can detect the peptide of the present invention. Since the peptide of the present invention is presented on an antigen-presenting cell, in particular a professional antigen-presenting cell such as a dendritic cell, the detecting agent is also useful for detecting an antigen-presenting cell that presents the peptide of the present invention.

In the present invention, the 'TCR-like antibody' is a molecule having a TCR-like binding force (antigen-recognizing ability) toward a complex (pMHC) between a fragmented antigen-derived peptide and a major histocompatibility complex (MHC) molecule. For example, as reported in Eur J Immunol. 2004; 34: 2919-29, etc., a TCR-like antibody that recognizes a complex between a tumor antigen-derived peptide and an MHC can recognize a cancer cell that is presenting a tumor antigen peptide that can be targeted by a CTL, a dendritic cell that has phagocytized a cancer cell and is presenting a tumor antigen peptide on an MHC class I, etc.

Furthermore, the TCR-like antibody that recognizes a complex between an MHC and a peptide derived from a virus, etc. can quantitatively and chronologically analyze what kind of presentation kinetics, CTL response, etc. a presented antigen will show on an infected cell.

The TCR-like antibody may be prepared by a method described in Eur J Immunol. 2004; 34: 2919-29, etc. For example, immunizing an animal such as a mouse with an MHC-peptide complex enables an antibody that is specific to the complex to be obtained. It is also possible to obtain a complex-specific antibody by utilizing a phage display method.

### (9) Tumor detection method (test method, diagnostic method)

The present invention provides a tumor detection method (test method, diagnostic method) utilizing the CTL-detecting agent or the tumor-detecting agent of the present invention.

The detection method (diagnostic method) of the present invention using the CTL-detecting agent of the present invention typically involves harvesting blood from a test subject or harvesting part of the test tissue for which a tumor is suspected by means of a biopsy, etc., and detecting/measuring the amount of CTLs that recognize a complex between an HLA antigen and an OR7C1-derived tumor antigen peptide contained therein by means of the CTL-detecting agent of the present invention, thus detecting, testing, or diagnosing the presence or absence or the extent of an OR7C1-positive cancer (tumor) such as colon cancer, lung cancer, or ovarian cancer.

The detection method (test method, diagnostic method) of the present invention using the tumor-detecting agent of the present invention typically involves harvesting blood from a test subject or harvesting part of the test tissue for which a tumor is suspected by means of a biopsy, etc., and detecting/measuring the amount of cells presenting a complex between an HLA antigen and an OR7C1-derived tumor antigen peptide contained therein by means of the tumor-detecting agent of the present invention, thus detecting, testing, or diagnosing the presence or absence or the extent of an OR7C1-positive cancer (tumor) such as colon cancer, lung cancer, or ovarian cancer.

For example, the detection (test, diagnostic) method of the present invention can detect (test, diagnose) the presence or absence or the extent of improvement of a tumor when a therapeutic drug is administered to a patient having a tumor in order to improve the tumor. Furthermore, the detection (test, diagnostic) method of the present invention may be applied to the selection of a cancer patient to whom a medicament containing the peptide or the polynucleotide of the present invention as an active ingredient can be applied effectively, and to the prediction, assessment, etc. of the therapeutic effect of the medicament. Moreover, in an embodiment in which the tumor-detecting agent of the present invention is used, it is possible to detect a cancer cell presenting a tumor antigen peptide that can be actually targeted by a CTL induced within the living body of a patient by administering a cancer vaccine containing the peptide of the present invention as an active ingredient.

A specific embodiment of the detection (test) method of the present invention using the CTL-detecting agent of the present invention includes steps (a) and (b), and optionally step (c), as follows:
(a) a step of bringing a biological sample obtained from a test subject into contact with the CTL-detecting agent of the present invention,
(b) a step of measuring the amount of CTLs that recognize a complex between an HLA antigen and an OR7C1-derived tumor antigen peptide in the biological sample using the amount of cells to which the CTL-detecting agent binds as an indicator, and
(c) a step of determining the presence of a cancer based on the result of (b).

A specific embodiment of the diagnostic method of the present invention using the CTL-detecting agent of the present invention includes steps (a), (b), and (c) above.

A specific embodiment of the detection (test) method of the present invention using the tumor-detecting agent of the present invention includes steps (d) and (e), and optionally step (f), as follows:
(d) a step of bringing a biological sample obtained from a test subject into contact with the tumor-detecting agent of the present invention,
(e) a step of measuring the amount of cells that present a complex between an HLA antigen and an OR7C1-derived tumor antigen peptide in the biological sample using the amount of cells to which the tumor-detecting agent binds as an indicator, and
(f) a step of determining the presence of a cancer based on the result of (e).

A specific embodiment of the diagnostic method of the present invention using the tumor-detecting agent of the present invention includes steps (d), (e), and (f) above.

Examples of the biological sample used here include a sample prepared from biological tissue (a tissue, surrounding tissue thereof, blood,etc. for which the presence of cancer cells is suspected) of a test subject. Specific examples include a sample containing peripheral blood lymphocytes prepared from the tissue.

One embodiment of the detection method (test method, diagnostic method) of the present invention using the CTL-detecting agent of the present invention is carried out by detecting a CTL specific to the peptide of the present invention in a biological sample and measuring the amount thereof. Specifically, a tetramer (HLA tetramer) of a complex between a fluorescently-labeled HLA antigen and the peptide of the present invention is prepared in accordance with a method described in the literature (Science, 274: p. 94, 1996, this publication forming part of the present application by reference), and this can be used for quantitatively determining by means of a flow cytometer the amount of antigen peptide-specific CTLs in peripheral blood lymphocytes of a patient for whom a cancer is suspected.

The prediction, assessment, determination, or diagnosis of the presence or absence of a tumor may be carried out by, for example, measuring the amount of CTLs specific to the peptide of the present invention in the blood or test tissue for which a tumor is suspected of a test subject or the amount of cells presenting the peptide of the present invention. In this process, in some cases, the level of OR7C1 gene expression, the level of the peptide of the present invention, or the level of CTLs, etc. in the corresponding normal tissue may be used as a reference value, and this reference value may be compared with the level in the sample obtained from the test subject, the difference between the two being assessed.

Here, the comparison of the levels between the test tissue of the test subject and the corresponding normal tissue may be carried out in parallel with measurement of the biological sample of the test subject and a biological sample of a healthy subject. When it is not carried out in parallel, the average value or the statistical median of the amounts of CTLs specific to the peptide of the present invention obtained using a plurality (at least two, preferably at least three, and more preferably at least five) of normal tissue pieces under uniform measurement conditions may be used in the comparison as the value for a healthy subject, that is, a reference value.

A determination of whether or not a test subject has a cancer may be carried out using as an indicator, for example, the amount of CTLs specific to the peptide of the present invention in tissue of the test subject or the cells presenting the peptide of the present invention being for example at least twice the level thereof in a healthy subject, and preferably at least three times.

Furthermore, in a test subject to which the peptide or the polynucleotide of the present invention is administered, it is also possible by measuring the amount of CTLs specific to the peptide of the present invention to assess whether or not CTLs have actually been induced. For example, it is possible to assess whether the treatment with the peptide or the polynucleotide of the present invention is effective by using as an indicator the amount of CTLs specific to the peptide of the present invention in the tissue of the test subject being for example at least twice the level thereof of a healthy subject, and preferably at least three times. Since it is considered that the peptide or the polynucleotide of the present invention mainly acts as a vaccine and induces CTLs to thus exhibit an anti-tumor action, it is possible to use the induction of CTLs as a POM (Proof of Mechanism) marker for confirming whether or not the peptide or the polynucleotide of the present invention administered acts as a vaccine. Therefore, by detecting CTLs as a POM marker, the CTL-detecting agent of the present invention can be used as a diagnostic agent for confirming whether or not a peptide or polynucleotide administered to a subject is acting as a vaccine.

### (10) Preventive and/or therapeutic method for cancer

The present invention also relates to a method for preventing and/or treating a cancer in a subject, the method including a step of administering an effective amount of an active ingredient selected from the group consisting of the peptide, the polynucleotide, the CTL, the antigen-presenting cell, and the TCR-like antibody of the present invention to a subject requiring same.

The 'subject' in the present invention means any biological individual, preferably an animal, more preferably a mammal, and more preferably a human individual. In the present invention, the subject may be healthy or may have any disease, but when the prevention and/or treatment of a cancer is intended, it typically means a subject having a cancer or having a risk thereof. In one embodiment of the present invention, the subject is HLA-A02 positive or HLA-A24 positive. In one embodiment of the present invention, the subject has an OR7C1-positive cancer or has a risk thereof. In one embodiment of the present invention, the subject is HLA-A02 positive or HLA-A24 positive and has an OR7C1-positive cancer or has a risk thereof.

With regard to the peptide, the polynucleotide, the CTL, the antigen-presenting cell, and the TCR-like antibody of the present invention used in the preventive/therapeutic method of the present invention, any one described in the present specification can be cited. The effective amount referred to in the present invention is an amount that for example reduces the symptoms of a cancer or delays or halts the progress thereof, and is preferably an amount that suppresses or cures a cancer. Furthermore, it is preferably an amount that does not cause an adverse effect that exceeds the benefit obtained by administration. Such an amount may be determined as appropriate by means of an in vitro test using cultured cells, etc. or a test in a model animal such as a mouse or a rat, and such test methods are well known to a person skilled in the art. The specific dose of an active ingredient may be determined while taking into consideration various conditions related to a subject requiring same, for example, the seriousness of symptoms, the general health state, age, and body weight of the subject, the sex of the subject, diet, timing and frequency of administration, concomitant medication, response to treatment, dosage form, compliance with treatment, etc.

In the case of for example the peptide of the present invention, the specific dose is usually 0.0001 mg to 1000 mg, preferably 0.001 mg to 1000 mg, and more preferably 0.1 mg to 10 mg, and this is preferably administered once in a few days to a few months. Furthermore, in the case of the polynucleotide of the present invention, it is usually 0.0001 mg to 100 mg, and preferably 0.001 mg to 10 mg, and this is administered once in a few days to a few months. In the case of the TCR-like antibody of the present invention, it is usually 0.0001 mg to 2000 mg, and preferably 0.001 mg to 2000 mg, and this is preferably administered once in 1 week to 4 weeks. As an administration method, any known appropriate administration method such as intradermal administration, subcutaneous administration, intramuscular administration, or intravenous administration may be used. It is also possible to use an in vivo method in which the peptide or the nucleotide of the present invention is directly administered into the body as well as an ex vivo method in which a specific type of cells are collected from a person, CTLs or antigen-presenting cells are induced in vitro using the peptide or the polynucleotide of the present invention, and these cells are subsequently returned into the body.

One embodiment of the preventive/therapeutic method of the present invention further includes, prior to the administration step, a step of screening a subject who is HLA-A02 positive or HLA-A24 positive as the subject for the prevention/treatment. This embodiment of the present invention may further include, prior to the selection step, a step of determining the HLA type of a subject. Determination of the HLA type of a subject may be carried out by any known method. Furthermore, one embodiment of the preventive/therapeutic method of the present invention further includes, prior to the administration step, a step of screening a subject having an OR7C1-positive cancer as a subject for the prevention/treatment. This embodiment of the present invention may further include, prior to the selection step, a step of detecting an OR7C1-positive cancer in a subject. Detection of an OR7C1-positive cancer in a subject may employ the tumor detection method described in (9) above.

One embodiment of the preventive/therapeutic method of the present invention further includes, prior to the administration step, a step of screening a subject who is HLA-A02 positive or HLA-A24 positive and has an OR7C1-positive cancer as a subject for the prevention/treatment. This embodiment of the present invention may further include, prior to the selection step, a step of determining the HLA type of a subject and a step of detecting an OR7C1-positive cancer in a subject. A subject who is HLA-A02 positive or HLA-A24 positive and has an OR7C1-positive cancer is a subject to whom a pharmaceutical composition containing the peptide and/or polynucleotide of the present invention as an active ingredient can be applied effectively. Therefore, the tumor-specific CTL-detecting agent or the tumor-detecting agent that can be used in the tumor detection method described in (9) above can be used as a diagnostic agent for a so-called companion diagnosis for screening a subject for treatment to whom a cancer treatment method using the pharmaceutical composition of the present invention is effective.

Furthermore, one embodiment of the preventive/therapeutic method of the present invention may further include a step of assessing whether or not the treatment with the pharmaceutical composition of the present invention is effective in a patient to whom the pharmaceutical composition of the present invention has been administered, and screening a subject for treatment for whom the treatment with the pharmaceutical composition of the present invention is effective. A described in (9) above, a subject in which CTLs specific to the peptide of the present invention are induced when the pharmaceutical composition of the present invention is administered is a subject for whom the treatment with a pharmaceutical composition containing the peptide and/or polynucleotide of the present invention as an active ingredient is effective, that is, a subject to whom the pharmaceutical composition of the present application can be applied effectively, and induction of CTLs specific to the peptide of the present invention may be used as a surrogate marker for predicting or assessing the therapeutic effect of the pharmaceutical composition of the present invention. Therefore, a tumor-specific CTL-detecting agent that can be used in the tumor detection method described in (9) above may be used as a diagnostic agent for screening an effective subject for treatment in patients to whom the pharmaceutical composition of the present invention has been administered or as a diagnostic agent for predicting or assessing the therapeutic effect of the pharmaceutical composition in a patient to whom the pharmaceutical composition of the present invention has been administered by detecting CTLs as the surrogate marker.

All patents, applications, and other publications referred to in the present specification are incorporated herein by reference in their entirety.

The present invention is specifically explained below by reference to Examples, but the present invention should not be construed as being limited by these Examples.

### [Examples]

### Example 1: OR7C1-derived peptide having HLA-A*02:01 binding motif

OR7C1-derived peptides for which were predicted to be bound to HLA-A*02:01 (peptides represented by SEQ ID Nos: 3, 4, 5, and 6) were selected by the use of BIMAS (http://www-bimas.cit.nih.gov/molbio/hla_bind/) and IEDB (MHC-I processing predictions; http://www.iedb.org/), which are programs for predicting the binding between an MHC and a peptide. These peptides were chemically synthesized by the Fmoc method and subjected to an HLA binding test and an in vivo CTL induction test.

### Example 2 : Evaluation of binding of OR7C1-derived peptide to HLA-A02 and HLA-A24

Evaluation of the binding of an OR7C1-derived peptide to HLA-A02 and HLA-A24 was carried out by an MHC class I expression stabilization test. In this test, T2 cells, which are a human lymphoblastoid cell line, and T2-A24 cells, which were artificially forced to express HLA-A*24:02, were used. T2 cells are deficient in the transporter associated with antigen processing (TAP), which is involved in the transport of a peptide from the cytoplasm to the endoplasmic reticulum. It is known that an MHC class I molecule (including HLA-A*02:01 and HLA-A*24:02) has an unstable structure in a state in which a peptide is not bound (empty MHC class I). Usually, T2 cells can only express a low level of empty MHC class I molecules on the cell surface. However, when a peptide that can bind to the MHC class I molecule is added, the empty MHC class I molecule binds to the peptide and can be present on the cell surface in a stable manner. Therefore, the cell surface MHC class I expression level depends on the MHC class I binding affinity of a peptide.

The T2 cells and the T2-A24 cells were subcultured at 37°C under 5% CO₂. With regard to peptides, an OR7C1-derived peptide (peptides described in Table 1), Melan A A27L peptide (amino acid sequence: ELAGIGILTV; SEQ ID No: 7) as an HLA-A02-positive control, HIV₅₈₄₋₅₉₂ peptide (amino acid sequence: RYLRDQQLL; SEQ ID No: 8) as an HLA-A24-positive control, MAGE-1₁₆₁₋₁₆₉ peptide (amino acid sequence: EADPTGHSY; SEQ ID No: 9) as an HLA-A02-negative control, and VSV₅₂₋₅₉ peptide (amino acid sequence: RGYVYQGL; SEQ ID No: 10) as an HLA-A24-negative control were each evaluated at a concentration of 100 µg/mL. These peptides were dissolved in DMSO and further diluted by 200 times with RPMI 160 medium. A cell suspension and the peptide solution were mixed and cultured under conditions of 5% CO₂ and 26°C for 16 to 18 hours. Co-culturing was carried out at a temperature of 37°C for a further 3 hours, the supernatant was then removed by centrifuging, and cells were isolated. The isolated cells were washed with PBS containing 3% FBS, an anti-HLA-A02 antibody (clone: BB7.2; Medical & Biological Laboratories Co., Ltd.) or anti-HLA-A24 antibody (clone: 17A10; Medical & Biological Laboratories Co., Ltd.) fluorescently-labeled with FITC was added, and the mixture was allowed to stand at room temperature for 30 minutes. Subsequently, the cells were washed with PBS containing 3% FBS, 4% paraformaldehyde phosphate buffer was added, and the mixture was allowed to stand at room temperature for 10 minutes to thus fix the cells. The fixed cells were subjected to measurement of FITC fluorescence intensity by a flow cytometer (FACScan). The mean fluorescence intensity (MFI) solvent ratio was calculated and a value of 1.5 or higher was determined to be positive.

The results are shown in Table 1. It can be seen from these results that all of the peptides represented by SEQ ID Nos: 3, 4, 5, and 6 show HLA-A*02:01 binding and HLA-A24 binding.

**[Table 1]**

| | SEQ ID No: | 3 | 4 | 5 | 6 |
|---|---|---|---|---|---|
| | Source | OR7C1 (117-126) | OR7C1 (276-285) | OR7C1 (118-126) | OR7C1 (34-44) |
| HLA-A02 Binding activity | Evaluation result | 2.2 | 2.5 | 2.3 | 2.6 |
| | Negative control | 1.0 | 1.0 | 1.0 | 1.0 |
| | Positive control | 3.7 | 3.7 | 3.7 | 3.7 |
| HLA-A24 Binding activity | Evaluation result | 2.0 | 2.4 | 2.4 | 5.1 |
| | Negative control | 1.0 | 1.0 | 1.0 | 1.1 |
| | Positive control | 1.9 | 1.9 | 1.9 | 4.3 |

### Example 3: Evaluation of in vivo CTL inducibility using HLA-A*02:01 transgenic mouse and HLA-A*24:02 transgenic mouse

The CTL inducibility of an OR7C1-derived peptide (SEQ ID No: 3, 4, 5 and 6) for which it had been determined that it bound to HLA-A*02:01 and HLA-A*24:02 was evaluated by an in vivo CTL induction test using an HLA-A*02:01 transgenic mouse and an HLA-A*24:02 transgenic mouse.

An HLA-A*02:01 transgenic mouse (C57BL/6CrHLA-A2.1DR1) is a mouse that is deficient in mouse MHC and expresses HLA-A*02:01 and HLA-DRB1*01:01, which are human MHCs, and the use of this mouse enables a peptide that can induce CTLs in humans to be selected. On the other hand, an HLA-A*24:02 transgenic mouse is a mouse that expresses a chimera HLA of HLA-A*24:02, which is a human MHC, and H-2K^{b}, which is a mouse MHC, and the use of this mouse enables a peptide that can induce CTLs in an HLA-A24-positive human to be selected (Int J Cancer. 2002; 100: 565-70). Whether or not CTLs are induced by administration of a peptide was determined by measuring the IFNγ produced when spleen cells derived from the mouse to which the peptide had been administered was restimulated with the administered peptide.

Specifically, the peptide was dissolved in PBS (-) at 10 mg/mL, and mixed with an equal part of incomplete Freund's adjuvant (IFA), thus forming an emulsion. The peptide thus emulsified was administered to the mouse tail base intradermally at two locations at a dose of 50 µg/location. One week after that, the mouse was euthanized with CO₂ gas, the spleen was removed, and spleen cells were prepared. For measurement of IFNγ production, an IFNγ ELISPOT assay kit (Becton, Dickinson and Company) was used. On the day before preparing the spleen cells, an ELISPOT plate was treated with an anti-IFNγ antibody, and on the day it was blocked with 10% FBS-containing RPMI 1640 medium. The spleen cells prepared were plated on the blocked ELISPOT plate at 0.5 × 10⁶ cells/well. The OR7C1-derived peptide was dissolved in DMSO at 40 mg/mL and further diluted with 10% RPMI 1640 medium to 20 µg/mL. The diluted peptide was added at 50 µL/well to the spleen cells derived from the animal to which the peptide had been administered. In vitro peptide restimulation was applied by culturing the spleen cells to which the peptide was added under 5% CO₂ at 37 °C for 16 to 18 hours. After culturing, the supernatant was removed, and the ELISPOT plate was subjected to coloration in accordance with the included protocol. The number of spots colored was measured using KS-ELISPOT.

The results of the IFNγ ELISPOT assay are shown in FIGs. 1, 2, 3, and 4.

It can be seen from the results of this test that, by confirming IFNγ production specific to each peptide in the HLA-A*02:01 transgenic mouse-derived and HLA-A*24:02 transgenic mouse-derived spleen cells, the OR7C1-derived peptides represented by SEQ ID Nos: 3, 4, 5, and 6 have CTL inducibility.

### Example 4: Evaluation of CTL inducibility using human peripheral blood mononuclear cells

An evaluation was carried out of whether or not CTLs were induced in healthy individual-derived peripheral blood mononuclear cells when three types of peptides represented by SEQ ID Nos: 3, 5, and 6 were added.

Specifically, peripheral blood mononuclear cells (Cellular Technology Limited) derived from an HLA-A*02:01-positive or HLA-A*24:02-positive healthy individual were suspended in AIM-V medium containing 10% human-derived serum, then plated in each well of a 96-well U-bottomed plate at 1 × 10⁵, and cultured at 37°C under 5% CO₂. In this process, 100 U/mL of human IL-2 and 20 µg/mL of the peptide above were added. The medium was exchanged every three or four days, and about 2 weeks later an IFNγ ELISPOT assay was carried out. On the day before the assay, the ELISPOT plate was treated with an anti-IFNγ antibody, and on the day it was blocked with AIM-V medium containing 10% human-derived serum at room temperature for about 2 hours. The human peripheral blood mononuclear cells during culturing were washed with AIM-V medium and plated on each cell of the blocked ELISPOT plate at one third of the amount. In this process, with regard to the human peripheral blood mononuclear cells cultured with the peptides represented by SEQ ID Nos: 3, 5, and 6, randomly selected two wells were mixed to give one well, each well was again divided and plated into two wells, the peptide solution was added to one of the wells, and a control solution not containing the peptide was added to the other well to give a non-restimulation control, 60 wells each being thus prepared. Here, three types of peptides represented by SEQ ID Nos: 3, 5, and 6 were dissolved in DMSO at 40 mg/mL and further diluted with AIM-V medium containing 10% human-derived serum. Addition was carried out so as to give a final concentration of 20 µg/mL, and the human peripheral blood mononuclear cells were restimulated. After culturing at 37°C under 5% CO₂ for 16 to 18 hours, the supernatant was removed, and the ELISPOT plate was subjected to coloration in accordance with the included protocol. The number of spots that had colored was counted using an ELISPOT analyzer from Cellular Technology Limited. The results of the IFNγ ELISPOT assay of SEQ ID Nos: 3, 5, and 6 are shown in FIG. 5, 6, and 7 respectively. The ordinate denotes the number of spots observed in each well and the abscissa denotes the positive wells. The black bar denotes the number of spots detected under peptide stimulation conditions and the white bar denotes the number of spots (control) detected under peptide non-pulse conditions. A positive well was determined only when the number of spots at the time of peptide stimulation was at least 50 and the difference between the black bar and the white bar was at least twice.

It can be seen from the results of this test that the three types of peptides represented by SEQ ID Nos: 3, 5, and 6 induce CTLs specific to these peptides from the peripheral blood mononuclear cells derived from an HLA-A*02:01-positive and HLA-A*24:02-positive healthy individual. It can be seen from this that these three types of peptides have CTL inducibility not only in mice but also in humans.

### Example 5: Evaluation of C terminal amino acid substituted peptide of OR7C1-derived peptide

### (1) Synthesis of peptide

A peptide (SEQ ID No: 11) for which valine at the C terminal amino acid of the peptide represented by SEQ ID No: 3 was replaced by isoleucine was chemically synthesized by the Fmoc method in the same manner as in Example 1 and subjected to an HLA binding test and an in vivo CTL induction test.

### (2)HLA-A02 and HLA-A24 binding evaluation

The peptide represented by SEQ ID No: 11 synthesized in (1) above was evaluated in the same manner as in Example 2 for binding to HLA-A02 and HLA-A24. The results are shown in Table 2. It can be seen from these results that the peptide represented by SEQ ID No: 11 shows HLA-A*02:01 binding and HLA-A24 binding.

**[Table 2]**

| | SEQ ID No: | 11 |
|---|---|---|
| | Source | OR7C1 V126I (117-126) |
| HLA-A02 Binding activity | Evaluation result | 2.2 |
| | Negative control | 1.0 |
| | Positive control | 3.6 |
| HLA-A24 Binding activity | Evaluation result | 3.8 |
| | Negative control | 1.0 |
| | Positive control | 2.4 |

### (3) Evaluation of in vivo CTL inducibility

CTL inducibility was evaluated by an in vivo CTL induction test using an HLA-A*02:01 transgenic mouse and an HLA-A*24:02 transgenic mouse as in Example 3. Specifically, it was carried out in the same manner as in Example 3 except that the peptide represented by SEQ ID No: 11 was dissolved in water for injection at 40 mg/mL, and mixed with an equal part of IFA, thus forming an emulsion, and the emulsified peptide was administered to the mouse tail base intradermally at two locations at a dose of 150 µg/location. The results are shown in FIG. 8.

It can be seen that the peptide represented by SEQ ID No: 11 has CTL inducibility since in the HLA-A*02:01 transgenic mouse-derived and HLA-A*24:02 transgenic mouse-derived spleen cells, production of IFNγ specific to the peptide was confirmed.

### (4) Evaluation of CTL inducibility using human peripheral blood mononuclear cells

An evaluation was carried out as in Example 4 of whether or not CTLs were induced by healthy individual-derived peripheral blood mononuclear cells when the peptide represented by SEQ ID No: 11 was added. Specifically, an evaluation was carried out in the same manner as in Example 4 except that, when human peripheral blood mononuclear cells cultured by the peptide represented by SEQ ID No: 11 were subjected to an IFNγ ELISPOT assay, without mixing any two wells to form one well, each well was divided into two wells and plated. The results are shown in FIG. 9. It can be seen that the peptide represented by SEQ ID No: 11 induces CTLs specific to the peptide from HLA-A*02:01-positive and HLA-A*24:02-positive healthy individual-derived peripheral blood mononuclear cells, and it has CTL inducibility not only in mice but also in humans.

### [Industrial Applicability]

In accordance with the present invention, there can be provided an OR7C1-derived tumor antigen peptide having CTL-inducing activity, etc. The peptide of the present invention is useful as an agent for the prevention and/or treatment of a cancer.

### [Sequence Table Free Text]

SEQ ID No: 3: synthetic peptide
SEQ ID No: 4: synthetic peptide
SEQ ID No: 5: synthetic peptide
SEQ ID No: 6: synthetic peptide
SEQ ID No: 7: synthetic peptide
SEQ ID No: 8: synthetic peptide
SEQ ID No: 9: synthetic peptide
SEQ ID No: 10: synthetic peptide
SEQ ID No: 11: synthetic peptide

## Claims

1. A peptide consisting of an amino acid sequence represented by any of SEQ ID No: 3, SEQ ID No: 4, SEQ ID No: 5, SEQ ID No: 6, and SEQ ID No: 11.

2. A peptide consisting of an amino acid sequence, the amino acid sequence being
(1) an amino acid sequence having an amino acid at the C terminal replaced by a hydrophobic amino acid, and/or
(2) an amino acid sequence having one to several amino acids added to the N terminal and/or the C terminal,
in any of SEQ ID No: 3, SEQ ID No: 4, SEQ ID No: 5, SEQ ID No: 6, and SEQ ID No: 11.

3. The peptide according to Claim 2, wherein an amino acid at the C terminal of the amino acid sequence represented by any of SEQ ID No: 3, SEQ ID No: 4, SEQ ID No: 5, SEQ ID No: 6, and SEQ ID No: 11 is replaced by leucine, valine, or isoleucine.

4. The peptide according to Claim 2, wherein one amino acid is added to the N terminal or the C terminal of the amino acid sequence represented by any of SEQ ID No: 3, SEQ ID No: 4, SEQ ID No: 5, SEQ ID No: 6, and SEQ ID No: 11.

5. A polyepitope peptide which comprises a plurality of epitope peptides linked together, wherein at least one of said epitope peptides is the peptide according to any one of Claims 1 to 4.

6. A pharmaceutical composition comprising as an active ingredient the peptide according to any one of Claims 1 to 4 or the polyepitope peptide according to Claim 5.

7. The pharmaceutical composition according to Claim 6, wherein the pharmaceutical composition further comprises an adjuvant.

8. A vaccine for the prevention and/or treatment of a cancer, the vaccine comprising as an active ingredient the peptide according to any one of Claims 1 to 4 or the polyepitope peptide according to Claim 5.

9. An agent for the prevention and/or treatment of a cancer, the agent comprising as an active ingredient the peptide according to any one of Claims 1 to 4 or the polyepitope peptide according to Claim 5.

10. An agent for inducing a cytotoxic T cell (CTL), the agent comprising as an active ingredient the peptide according to any one of Claims 1 to 4 or the polyepitope peptide according to Claim 5.

11. A polynucleotide encoding at least one of the peptide according to any one of Claims 1 to 4 or the polyepitope peptide according to Claim 5.

12. An expression vector comprising the polynucleotide according to Claim 11.

13. A composition for gene transfer, the composition comprising the expression vector according to Claim 12.

14. A pharmaceutical composition for the treatment or prevention of a cancer, comprising as an active ingredient either (a) or (b) below:
(a) the polynucleotide according to Claim 11
(b) the expression vector according to Claim 12.

15. A method for producing an antigen-presenting cell, comprising contacting in vitro a cell having an antigen-presenting ability with (a) or (b) below:
(a) the peptide according to any one of Claims 1 to 4 or the polyepitope peptide according to Claim 5,
(b) a polynucleotide encoding at least one of the peptides described in (a) above.

16. A method for inducing a CTL, comprising contacting in vitro a peripheral blood lymphocyte with either (a) or (b) below:
(a) the peptide according to any one of Claims 1 to 4 or the polyepitope peptide according to Claim 5,
(b) a polynucleotide encoding at least one of the peptides described in (a) above.

17. An HLA multimer comprising an HLA and the peptide according to any one of Claims 1 to 4.

18. A diagnostic agent comprising the HLA multimer according to Claim 17.

19. A TCR-like antibody that recognizes a complex between an HLA and the peptide according to any one of Claims 1 to 4.

20. A tumor-detecting agent comprising the TCR-like antibody according to Claim 19.

21. A diagnostic agent for screening a patient to whom the pharmaceutical composition according to Claim 6, 7, or 14 can be applied effectively, the diagnostic agent comprising the HLA multimer according to Claim 17 or the TCR-like antibody according to Claim 19.


SEQUENCE LISTING

<110>  Sumitomo Dainippon Pharma Co., Ltd.
       Sapporo Medical University

<120>  Tumor antigenic peptide
<130>  2652DN

<150>  JP2013-146849
<151>  2013-07-12

<160>  11

<170>  PatentIn version 3.5

<210>  1
<211>  963
<212>  DNA
<213>  Homo sapiens

<400>  1
   atggaaacag gaaatcaaac acatgcccaa gaatttctcc tcctgggatt ttcagcaacg      60
   tcagagattc agttcattct ctttgggctg ttcctctcca tgtacctagt cactttcacc     120
   gggaacctgc tcatcatcct ggccatatgc tcagactccc acctccacac ccccatgtac     180
   ttcttcctct ccaacctgtc ttttgctgac ctctgtttta cctccacgac tgtcccaaag     240
   atgttactga atatactgac acagaacaaa ttcataacat atgcaggctg tctcagtcag     300
   attttttttt tcacttcatt tggatgcctg gacaatttac tcttgaccgt gatggcctat     360
   gaccgcttcg tggccgtctg tcaccccctg cactatacgg tcatcatgaa cccccagctc     420
   tgtggactgc tggttctggg gtcctggtgc atcagtgtca tgggttccct gctcgagacc     480
   ttgactgttt tgaggctgtc cttctgcacc gaaatggaaa ttccacactt tttttgtgat     540
   ctacttgaag tcctgaagct cgcctgttct gacaccttca ttaataacgt ggtgatatac     600
   tttgcaactg gcgtcctggg tgtgatttcc ttcactggaa tatttttctc ttactataaa     660
   attgttttct ctatactgag gatttcctca gctgggagaa agcacaaagc gttttccacc     720
   tgtggttccc acctctcagt ggtcaccttg ttctatggca cgggctttgg ggtctatctc     780
   agttctgcag ccacaccatc ttctaggaca agtctggtgg cctcagtgat gtacaccatg     840
   gtcaccccca tgctgaaccc cttcatctac agcctgagga acacggacat gaagagggcc     900
   ctggggagac tcctcagtag ggcaacattt tttaatggtg acatcactgc aggactttca     960
   taa                                                                   963


<210>  2
<211>  320
<212>  PRT
<213>  Homo sapiens

<400>  2

Met Glu Thr Gly Asn Gln Thr His Ala Gln Glu Phe Leu Leu Leu Gly
1               5                   10                  15

Phe Ser Ala Thr Ser Glu Ile Gln Phe Ile Leu Phe Gly Leu Phe Leu
            20                  25                  30

Ser Met Tyr Leu Val Thr Phe Thr Gly Asn Leu Leu Ile Ile Leu Ala
        35                  40                  45

Ile Cys Ser Asp Ser His Leu His Thr Pro Met Tyr Phe Phe Leu Ser
    50                  55                  60

Asn Leu Ser Phe Ala Asp Leu Cys Phe Thr Ser Thr Thr Val Pro Lys
65                  70                  75                  80

Met Leu Leu Asn Ile Leu Thr Gln Asn Lys Phe Ile Thr Tyr Ala Gly
                85                  90                  95

Cys Leu Ser Gln Ile Phe Phe Phe Thr Ser Phe Gly Cys Leu Asp Asn
            100                 105                 110

Leu Leu Leu Thr Val Met Ala Tyr Asp Arg Phe Val Ala Val Cys His
        115                 120                 125

Pro Leu His Tyr Thr Val Ile Met Asn Pro Gln Leu Cys Gly Leu Leu
    130                 135                 140

Val Leu Gly Ser Trp Cys Ile Ser Val Met Gly Ser Leu Leu Glu Thr
145                 150                 155                 160

Leu Thr Val Leu Arg Leu Ser Phe Cys Thr Glu Met Glu Ile Pro His
                165                 170                 175

Phe Phe Cys Asp Leu Leu Glu Val Leu Lys Leu Ala Cys Ser Asp Thr
            180                 185                 190

Phe Ile Asn Asn Val Val Ile Tyr Phe Ala Thr Gly Val Leu Gly Val
        195                 200                 205

Ile Ser Phe Thr Gly Ile Phe Phe Ser Tyr Tyr Lys Ile Val Phe Ser
    210                 215                 220

Ile Leu Arg Ile Ser Ser Ala Gly Arg Lys His Lys Ala Phe Ser Thr
225                 230                 235                 240

Cys Gly Ser His Leu Ser Val Val Thr Leu Phe Tyr Gly Thr Gly Phe
                245                 250                 255

Gly Val Tyr Leu Ser Ser Ala Ala Thr Pro Ser Ser Arg Thr Ser Leu
            260                 265                 270

Val Ala Ser Val Met Tyr Thr Met Val Thr Pro Met Leu Asn Pro Phe
        275                 280                 285

Ile Tyr Ser Leu Arg Asn Thr Asp Met Lys Arg Ala Leu Gly Arg Leu
    290                 295                 300

Leu Ser Arg Ala Thr Phe Phe Asn Gly Asp Ile Thr Ala Gly Leu Ser
305                 310                 315                 320

<210>  3
<211>  10
<212>  PRT
<213>  Artificial

<220>
<223>  synthetic peptide

<400>  3

Val Met Ala Tyr Asp Arg Phe Val Ala Val
1               5                   10

<210>  4
<211>  10
<212>  PRT
<213>  Artificial

<220>
<223>  synthetic peptide

<400>  4

Val Met Tyr Thr Met Val Thr Pro Met Leu
1               5                   10

<210>  5
<211>  9
<212>  PRT
<213>  Artificial

<220>
<223>  synthetic peptide

<400>  5

Met Ala Tyr Asp Arg Phe Val Ala Val
1               5

<210>  6
<211>  11
<212>  PRT
<213>  Artificial

<220>
<223>  synthetic peptide

<400>  6

Met Tyr Leu Val Thr Phe Thr Gly Asn Leu Leu
1               5                   10


<210>  7
<211>  10
<212>  PRT
<213>  Artificial

<220>
<223>  synthetic peptide

<400>  7

Glu Leu Ala Gly Ile Gly Ile Leu Thr Val
1               5                   10


<210>  8
<211>  9
<212>  PRT
<213>  Artificial

<220>
<223>  synthetic peptide

<400>  8

Arg Tyr Leu Arg Asp Gln Gln Leu Leu
1               5


<210>  9
<211>  9
<212>  PRT
<213>  Artificial

<220>
<223>  synthetic peptide

<400>  9

Glu Ala Asp Pro Thr Gly His Ser Tyr
1               5


<210>  10
<211>  8
<212>  PRT
<213>  Artificial

<220>
<223>  synthetic peptide

<400>  10

Arg Gly Tyr Val Tyr Gln Gly Leu

1               5

<210>  11
<211>  10
<212>  PRT
<213>  Artificial

<220>
<223>  synthetic peptide

<400>  11

Val Met Ala Tyr Asp Arg Phe Val Ala Ile
1               5                   10